Europäisches Patentamt

European Patent Office

Office européen des brevets

(19)

(11) Publication number: **0 274 219**
**A2**

(12)

# EUROPEAN PATENT APPLICATION

(21) Application number: 87310653.8

(51) Int. Cl.⁴: **A61K 37/02** , A61K 9/50

(22) Date of filing: 03.12.87

The applicant has filed a statement in accordance with Rule 28 (4) EPC (issue of a sample only to an expert). Accession number(s) of the deposit(s): IFO 14437 - FERM BP-852.

(30) Priority: 04.12.86 JP 289449/86
09.06.87 JP 144489/87

(43) Date of publication of application:
13.07.88 Bulletin 88/28

(84) Designated Contracting States:
AT BE CH DE FR GB IT LI LU NL SE

(71) Applicant: **Takeda Chemical Industries, Ltd.
27, Doshomachi 2-chome Higashi-ku
Osaka-shi Osaka, 541(JP)**

(72) Inventor: **Sato, Jun
19-301, 5, Aoki-cho
Nishinomiya Hyogo 662(JP)**
Inventor: **Uda, Yoshiaki
2-501, 3 Nikawadanchi
Takarazuka Hyogo 665(JP)**
Inventor: **Fukuta, Makoto
19-406, 11 Toyosato 7-chome
Higashiyodogawa-ku Osaka 533(JP)**

(74) Representative: **Laredo, Jack Joseph et al
Elkington and Fife High Holborn House 52/54
High Holborn
London, WC1V 6SH(GB)**

(54) Interleukin-2-containing liposome composition and method for the production thereof.

(57) Interleukin-2 in an interleukin-2-containing liposome composition in which the pH of the interleukin-2-containing buffer solution to be sealed in liposome is adjusted 2 to 3.5 is stable for a long time.

EP 0 274 219 A2

# INTERLEUKIN-2-CONTAINING LIPOSOME COMPOSITION AND METHOD FOR THE PRODUCTION THEREOF

The present invention is directed to interleukin-2-containing liposome composition and method for the production thereof.

Interleukin-2 (hereinafter sometimes abbreviated to IL-2) is a protein that functions as a growth factor for T-cells and natural killer cells, both of which play a critical role in immunoregulation *in vivo* and which are thought of as directly or indirectly acting for cancer elimination, recovery from immunodeficiency, and immunopotentiation [Nature, vol. 302, pp. 305-310 (1983)]. Since possessing such bioactivities, human IL-2 is much expected to be applied for novel therapeutic drugs for immunodeficiency or anitcancer agents.

As liposome compositions are known multilamella vesicles (MLV), small unilamella vesicles (SUV), large unilamella vesicles (LUV) and reverse-phase evaporation vesicles (REV); methods of their production are described in, for example, "Saibo Kogaku", (Cell Engineering), vol. 2, No.9, pp.1136-1150 (1983). A liposome named stable plurilamellar vesicle has recently become known (Japanese Published Unexamined Patent Application No. 500952/1984, PCT International Publication No. WO 83/03383). An IL-2-containing liposome as decribed in Japense Patent Laid-open No. 243022/1985 (which corresponds to UK Patent Application GB 2157172A) is also known.

In the above-mentioned Japanese Patent Laid-open No. 243022/1985 (which corresponds to UK Patent Application GB 2157172A), it is stated that it is preferable that, when IL-2 is sealed in liposome, the pH of the buffer be from 4 to 7.

However, when examined for residual IL-2 activity, an IL-2 containing liposome prepared using a buffer which had been adjusted to pH of not less than 4 was found to decrese its activity.

Based on the above-mentioned results, the present inventors made investigations to obtain interleukin-2-containing liposome composition that could contain IL-2 under stable conditions, and found that the said purpose is accomplished by using a buffer solution that has been adjusted to pH of 2 to 3.5. Based on this finding, the present inventors make further investigations, thus completing the present invention.

The present invention involves:

(1) an interleukin-2-containing liposome composition in which the pH of the interleukin-2-containing buffer solution to be sealed in liposome is 2 to 3.5;

(2) a method of producing interleukin-2-containing liposome composition by the hydration of membranous phospholipid using an interleukin-2-containing buffer soltuion, which comprises adjusting the said buffer solution of pH of 2 to 3.5; and

(3) a method of producing interleukin-2-containing liposome composition by mixing together a phospholipid solution and an interleukin-2-containing buffer solution to prepare a W/O type emulsion, gelating this emulsion, then adding a buffer to the resulting gel, which comprises adjusting at least the buffer to be added to the gel to pH of 2 to 3.5.

For the above-mentioned IL-2, any substance can be used, as long as it possesses IL-2 activity, i.e., the activity by which T-cells can be subcultured. As examples of such substances, mention may be made of natural IL-2 produced in animal bodies and cells, IL-2 produced by genetic engineering technique, and related substances. These IL-2s and related substances may, or may not, have a sugar chain.

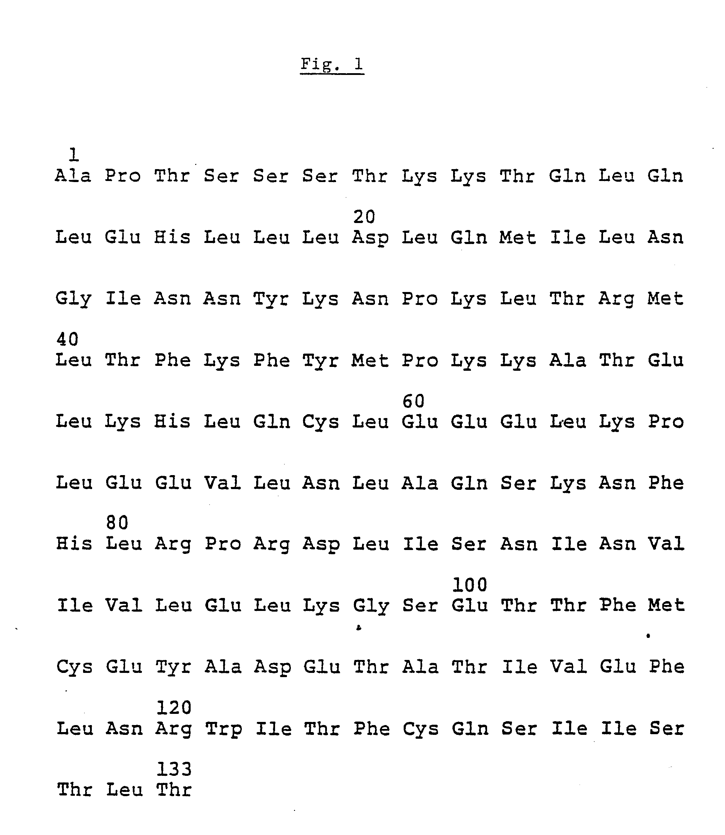

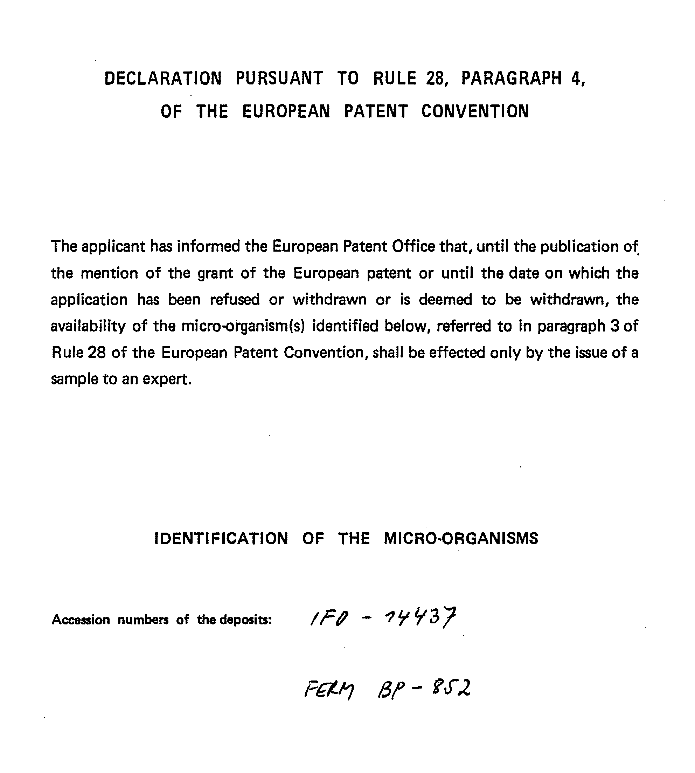

As specific examples of such IL-2s, mention may be made of the polypeptide (I) having the amino acid sequence shown in Figure 1, produced by genetic engineering techniques [Japanese Patent Laid-open No. 78799/1986 which corresponds to European Patent Publication No. 176299] and those fragments consisting of a partial amino acid sequence essential to the biological or immunological activity thereof. As examples of such fragments, mention may be made of the fragments resulting from the deletion of 1 amino acid (European Patent Publication No. 91539) or 4 amino acids (Japanese Patent Laid-open No. 126088/1985) in the amino terminal of the polypeptide (I) and those fragments resulting from the deletion of several amino acids in the carboxyl terminal of the polypeptide (I). Those fragments resulting from the deletion, or subsitution by other amino acids, or some constitutional amino acids in the polypeptide (I), e.g., the fragment resulting from the substitution of the 125-cysteine residue by a serine residue (Japanese Patent Laid-open No. 93093/1984 which corresponds to U.S. Patent No. 4,518,584) may also be used.

The above IL-2s may be chemically modified with polyethylene glycol derivatives [e.g. Japanese Patent Laid-open No. 226821/1985 which corresponds to European Patent Publication No. 154316].

The polypeptide (I) may have additional *Met* at the amino terminal [Japanese Patent Laid-open No. 78799/1986 which corresponds to European Patne Publication No. 176299].

Mixtures of the polypeptide (I) and the polypeptide(I) having additional *Met* at the amino terminal may also be used [Japanese Patent Laid-open No. 115528/1985 which corresponds to European Patent Publication No. 145390].

Well-known techniques are used to produce the IL-2 liposome composition of the present invention.

As to a method of their production, (A) multilamella vesicles (MLV) can be otained by evaporating off the solvent from a phospholipid solution to prepare a phospholipid film, then hydrating the resulting membranous phospholipid using an IL-2-containing buffer solution. (B) Small unilamella vesicles (SUV) can be obtained by shaking the multilamella vesicles thus obtained using an ultasonic shaker. The present invention resides in that the IL-2-containing buffer solution used in the above-mentioned process (A) or (B) has been adjusted to pH of 2 to 3.5.

As to another method of producing the IL-2 liposome preparations of the present invention, (C) reverse-phase evaporation vesicles (REV) or large unilamella vesicles (LUV) can be obtained by mixing togher a phospholipid solution and an IL-2-containing buffer solution to prepare a W/O type emulsion, evaporating off the solvent of this emulsion to gelate the emulsion, then adding a buffer to the resulting gel. (D) Stable plurilamellar vesicles (SPLV) can be obtained by mixing together a phospholipid solution and an IL-2-containing buffer soltuion, carrying out solvent evaporation and ultrasonication simultaneously to obtain a gel, then adding a buffer to this gel. The present invention resides in that at least the buffer solution to be added to the gel in the above-mentioned process (C) or (D) has been adjusted to pH of 2 to 3.5.

For the phospholipid used to produce the liposome preparations of the present invention, *per se* known phospholipids can be used, including egg yolk lecithin, soybean lecithin, partially hydrogenated products thereof, semi-synthetic phospholipids (e.g. phosphatilcholine, phosphatidylethanolamine, and so forth, which are semi-synthetically obtained from combination of lauryl, myristoyl, palmitoyl, stearoyl, oleyl and so forth.), phosphatidylserine, phosphatidylglycerol, sphingomyelin, and so forth.

Phosphatidic acid may also be mentioned as a phospholipid.

The above-mentioned phospholipids may be used alone, or in any combination of two or more of them. They may also be used in mixture with lipid constituents other than phospholipid, such as acylated mono-, di-, and triglycerides and cholesterol.

For the liposome stabilization, cholesterol, dicetyl phosphate, stearylamine, antioxidants, BHT (butylhydroxytoluene), $\alpha$-tocopherol and the like may also be added.

It is preferable that the mixing ratio of IL-2 to phospholipid be about 50 to 200 mg of Il-2 to about 50 to 10,000 $\mu$g of phospholipid. It is preferable that IL-2 be present in an amont of about 300 to 5,000 $\mu$g.

For the present invention, any buffer can be used, as long as it is capable of aiding in producing liposome preparations. As examples of such buffers, mention may be made of phosphate buffers, citrate buffers, lactate buffers, acetate buffers and ammonium acetate solutions, adjusted to suitable pH with, for example, hydrochloric acid.

It is preferable that the buffer concentration of the buffer solution be about 5mM to 50mM.

It is preferable that the IL-2 concentration of IL-2-containing buffer solution for the present invention be about 0.5 to 10 mg/m$\ell$, calculated on the IL-2 protein basis, and the use of a concentration of about 0.5 to 5 mg/m$\ell$ is more practical.

In producing the interleukin-2-containing liposome composition of the present invention, the buffer is adjusted to pH of 2 to 3.5.

It is more preferable that the buffer for the present invention be adjusted to pH about 3.

Buffer pH is adjusted by the addition of, for example, hydrochlorid acid, to the IL-2 solution in, for example, a 5mM ammonium acetate solution.

In the process (D) or (E), the IL-2-containing buffer solution to be mixed with the phospholipid solution may be be adjusted to pH of 2 to 3.5; however, it is normally recommended that the buffer solution be adjusted to pH of about 2 to 3.

Various additives may be added to this IL-2-containing buffer solution, e.g., human serum albumin or antioxidant as stabilizers or antiadsorbents.

Solvents which can be used for the phospholipid solution in the above-mentioned processes (A) and (B) include ether, chloroform and isopropylether. These solvents may be mixed with each other before use.

For solvent evaporation, ordinary procedures including rotary evaporation can be employed. An N$_2$ gas flow may be used to remove the residual organic solvent.

For hydration in the above-mentioned processes (A) and (B), ordinary methods can be employed; for example, the buffer is added, then the subject material is kept standing at room temperature for about 1 to 24 hours.

For emulsion preparation in the above-mentioned process (C), a W/O type emulsion is prepared by probe-type ultrasonication.

This ultrasonication is achieved, for example, by repeating 3 to 10 times at certain intervals the cycle in which ultrasound is applied for about 10 to 60 consecutive seconds, which is followed by non-treatment period.

For emulsion solvent evaporation in the above-mentioned process (C), ordinary methods including rotary evaporation can be employed. An N₂ gas flow may be used to remove the residual organic solvent. As to the method of buffer addition to the gel, the buffer is added directly. Liposome can be thus obtained.

For simultaneous solvent evaporation and ultrasonication in the above-mentioned process (D), a W/O type emulsion of the organic solvent containing dissolved phospholipid and the buffer solution containing dissolved IL-2 is produced by ultrasonication, while an N₂ gas flow is applied slowly to this emulsion so that the organic solvent is removed. A gel can be thus obtained. Liposomes are obtained by agitation and shaking following the addition of a buffer to the resulting gel.

For collecting the liposomes thus obtained, centrifugation at about 8,000 to 10,000 g for about 5 to 20 minutes is normally used.

As to the method of storing the IL-2 liposome compositions of the present invention, the liposome compositions are sealed in containers in which air has been replaced by N₂ gas, and stored in cool, dark place.

Since stably holding IL-2 for a long period, the IL-2 liposome compositions of the present invention exhibit persistant therepeutic effect of IL-2. The IL-2 liposome compositions of the present invention can therefore work well as sustained release compositions.

The IL-2 liposome compositions of the present invention can be used suitably as parenteral preparations.

Such parenteral preparations include preparations for injection.

When the IL-2 liposome compositions of the present invention are to be used as preparations for injection, the obtained liposome dispersion is used directly, or dispersed, as thick liposome, in a dispersant [e.g., a physiological saline, an isotonized aqueous solution (containg mannitol or the like)] before use, or, where possible, lyophilized, then re-dispersed before use. The preparations for injection thus obtained may be administered subcutaneously or intramuscularly.

Due to their low toxicity the IL-2 liposome compositions of the present invention can be used for the same purposes and subjects, and in the same dosages and uses, as the well-known IL-2.

The IL-2s employed in the present invention are low in toxicity. For example, the IL-2 produced by genetic engineering technique, purified by the procedure described in Japanese Patent Laid-open No. 115528/1985 (which corresponds to European Patent Publication No. 145390) and isolated by the procedure described in Japanese Patent Laid-open No. 78799/1986 (which corresponds to European Patent Publication No. 176299), whose amino acid sequence is as shown in Fig.1 and whose specific acitivity is about $3.5 \times 10^4$ units/mg, shows no fatal toxicity when it is administered to cynomolgus monkeys intravenously in a single dose of 6 mg/kg.

As stated above, IL-2 active substances are low in toxicity, and therefore they can be adminstered safely.

When administered in the liposome compostions of the present invention, the base agent IL-2 is released gradually, that is a persistent therapeutic effect of IL-2 is expected. It is therefore preferable that the liposome composition containing IL-2 in an amount corresponding to the dose for from several days to several weeks be administered, for example, in an amount corresponding to the dose for from 1 to 2 weeks. When administered parenterally in an amount corresponding to the dose for 1 week, the liposome composition is administered in such amount that IL-2 is administered at a dose of about 10 to 100 U/kg; when administered in an amount corresponding to the dose for 2 weeks, the liposome composition is administered in such amount that IL-2 is administered at a dose of about 20 to 200 U/kg.

1U (unit) is the arbitrarily determined IL-2 activity of 1 mℓ of a standard sample, corresponding to about 28.6 ng of pure recombinant IL-2.

The assay of IL-2 activity and the definition of IL-2 activity unit are as set forth in Japanese Patent Laid-open No. 115528/1985 which corresponds to European Patent Publication No. 145390.

The IL-2 liposome compositions of the present invention can be used for the prevention and treatment of immunological diseases and tumors in mammals (e.g., mice, cats, dogs, bovines, horses, sheep, goats, rabbits, and humans).

Since IL-2 sealed in the liposome composition remains stable for a long time, the IL-2-containing liposome composition of the present invention can be used as sustained release preparations.

Brief Description of the Drawing

Figure 1 presents the amino acid sequence of human IL-2 whose amino terminal is H-Ala-.

The present ivention is hereinafter described in more detail with some examples, but which are not to be construed as limitations thereof.

The IL-2 used in the examples is a mixture of IL-2 whose amino terminal begins with alanine [polypeptide (I)] and the IL-2 resulting from the addition of methionine to the N-terminal of the polypeptide (I), and has a specific activity of about $3.5 \times 10^4$ units/mg obtained by the method described in Reference Example 2 of Japanese Patent Laid-open No. 78799/1986 (which corresponds to European Patent Publication No. (176299) using the transformant Escherichia coli N4830/pTB285 (IFO 14437, FERM BP-852). The transforrmant Eschereichia coli N4830/pTB285 has been deposited at the Institute for Fermentation, Osaka (IFO), Japan under the accession number of IFO 14437 since April 25, 1985. An the transformant has been also deposited at the Fermentation Research Institute, Agency of Industrial Science and Technology, Ministry of International Trade and Industry (FRI), Japan under the accession number of FERM P-8199 since April 30, 1985, and the deposit has been converted to the deposit under the Budapest Treaty and the transformant has been stored at FRI under the accession number of FERM BP-852.

Example 1

1 mℓ of a 100 mg/mℓ solution of egg yolk lecithin (EPC) in chloroform was added to a 50 to 100 mℓ round-bottom flask. The chloroform was evaporated off using a rotary evaporator to obtain an EPC film, which was then dissolved in 5 mℓ diethyl ether. To the resulting solution of EPC in diethyl ether was added 0.3 mℓ of an acetate buffer solution containing 1.0 mg/mℓ IL-2, adjusted to pH of 3.0 with hydrochloric acid. This round-bottom flask was subjected to ultrasonication using a bathtub-type ultrasound generator (60W, 45KHz), and the diethyl ether was evaporated in a slow nitrogen gas flow so that a gel containing both IL-2 and EPC was produced. The gel produced on the inside wall of the round-bottom flask was suspended in 10 mℓ of an ammonium acetate buffer which has been adjusted to pH of 3 with hydrochloric acid to obtain liposomes containing sealed IL-2. The IL-2 not sealed in liposome was removed from the liposome suspension by centrifugation (10,000 g X 10 minutes) to separate the IL-2 liposome preparation.

Example 2

IL-2 liposome preparations were produced in the same manner as in Example 1 except that the buffer solution added to the gel had been adjusted to pH of 2 or 3.5.

Example 3

1 mℓ of a 100 mg/mℓ solution of egg yolk lecithin (EPC) in chloroform was added to a 100 mℓ round-bottom flask. The chloroform was evaporated off using a rotary evaporator to obtain an EPC film, which was then dissolved in 5 mℓ diethyl ether. To the resulting solution of EPC in diethyl ether was added 0.3 mℓ of an ammonium acetate buffer solution containing 1.0 mg/mℓ IL-2 adjusted to pH of 5 with hydrochloric acid. This round-bottom flask was subjected to ultrasonication using a bathtub-type ultrasound generator (60W, 45KHz), and the diethyl ether was evaporated in a slow nitrogen gas flow so that a gel containing both IL-2 and EPC was produced. The gel produced on the inside wall of the round-bottom flask was suspended in 10 mℓ of an ammonium acetate buffer which had been adjusted to pH of 3 with hydrochloric acid to obtain liposomes containing sealed IL-2. The IL-2 not sealed in liposome was removed from the liposome suspension by centrifugation (10,000 g X 10 minutes) to separate a liposome preparation containing sealed IL-2.

Example 4

1 mℓ of a 100 mg/mℓ solution of egg yolk lecithin (EPC) in chloroform was added to a 100 mℓ round-bottom flask. The chloroform was evaporated using a rotary evaporator to obtain an EPC film. To this flask was added 2 mℓ of an ammonium acetate buffer solution containing 1.0 mg/mℓ IL-2 adjusted to pH of 3

5

with hydrochloric acid. After complete hydration, the mixture was shaken until the EPC film was suspended completely. The resulting suspension, after being thrice washed with 10 mℓ of an ammonium acetate buffer adjusted to pH of 3 with hydrochloric acid, was centrifuged to remove IL-2s not sealed in liposomes, and an IL-2 liposome preparation was separated.

Example 5

3 mℓ pf a 100 mg/mℓ solution of egg yolk lecithin (EPC) in chloroform was added to a 500 mℓ round-bottom flask. The chloroform was evaporated using a rotary evaporator to obtain an EPC film, which was then dissolved in 70 mℓ diethyl ether. To this solution was added 10 mℓ of an ammonium acetate buffer solution containing 1.0 mg/mℓ IL-2 adjusted to pH of 3 with hydrochloric acid, and this was followed by emulsification using a probe-type ultrasonic shaker (75W, 20KHz, 30 seconds X 10 times) to prepare a W/O type emulsion. The emulsion thus obtained was transferred into a rotary evaporator, then the diethyl ether was evaporated off under reduced pressure to obtain a gel, which was suspended in 10 mℓ of an ammonium acetate buffer adjusted to pH of 3 with hydrochloric acid. From the resulting suspension IL-2 not sealed was removed, and an IL-2 liposome preparation was separated.

Reference example 1

An IL-2 liposome preparation was produced in the same manner as in Example 1 except that the buffer solution added to the gel had been adjusted to pH of 1, 4, or 5.

Experimental example 1

The IL-2 liposomes preduced in Examples 1 and 2 and Reference Example 1 were suspended in physiological saline, and the stabilities of IL-2 in the liposomes were examined. A combination of a bulk solution of the pH 1.5 and a suspension buffer of the pH of 1.5 was used as control.

10 mℓ of each liposome suspension was kept standing at 37°C, during which IL-2 on the liposome suspension was determined by HPLC at the specified intervals. In the determination by HPLC, IL-2 was separated by the gradient method, then it was determined by fluorescence detection. The flow rate was 2 mℓ, and a 0.1% aqueous solution of trifluoroacetic acid (eluent-A) and a 0.1% solution of trifluoroacetic acid in acetonitrile (eluent-B) were used as gradient eluents. Over a period of 15 minutes, a linear gradient of from 40% to 65% of the eluent-A was made. Fluorescence was detected at an excitation wavelength of 280 nm and an emission wavelength of 340 nm. IL-2 was separated in approximately 11 minutes. The column used was Ultron 300C4 (4.60 X 150 nm). The results are shown in Table 1.

Table 1    Rates of Residual IL-2 in Liposomes

| Time elapsed | pH of added IL-2 bulk buffer solution and gel suspension buffer | | | | | | | |
|---|---|---|---|---|---|---|---|---|
| | pH1 | pH1.5 | pH2 | pH3 | pH3.5 | pH3.75 | pH4 | pH5 |
| 0 day | 100% | 100% | 100% | 100% | 100% | 100% | 100% | 100% |
| 1 day | 76 | 86 | 94 | 95 | 85 | 81 | 77 | 41 |
| 7 days | 0 | 31 | 93 | 90 | 71 | 65 | 53 | 20 |

As shown in Table 1, when the added IL-2 bulk buffer solution and the gel suspension buffer were adjusted to pH of 2, 3, or 3.5, the stability of IL-2 in liposome was improved noticeably.

Experimental example 2

The IL-2 liposome produced in Example 3 was suspended in physiological saline, and the stability of IL-2 in the liposome was examined. A combination of an IL-2 bulk buffer solution of pH 5 and a gel suspension buffer of pH 5, both of which were prepared in the same manner as in Example 3, was used as control. 10 mℓ of the liposome suspension was kept standing at 37°C, during which IL-2 in the liposome suspension was determined by HPLC at the specified intervals, as shown in Example 2. The results are shown in Table 2.

Table 2    Rates of Residual IL-2 in Liposomes

| Time elapsed (days) | Control liposome | Liposome obtained in Example 2 |
|---|---|---|
| 0 | 100% | 100% |
| 1 | 41 | 97 |
| 7 | 20 | 70 |

As shown in Table 2, when the gel suspension buffer was adjusted to pH of 3, the stability of IL-2 in liposome was improved noticeably.

**Claims**

1. An interleukin-2-containing liposome composition in which the pH of the interleukin-2-containing buffer solution to be sealed in liposome is adjusted to 2 to 3.5.

2. A composition as claimed in Claim 1, wherein the interleukin-2 is a recombinant non-glycosylated human interleukin-2.

3. A composition as claimed in claim 1, wherein the pH is about 3.

4. A method of producing interleukin-2-containing liposome composition by the hydration of membranous phospholipid using an interleukin-2-containing buffer solution, which comprises adjusting the pH of the said buffer solution of 2 to 3.5.

5. A method as claim 4, wherein the interleukin-2 is a recombinant non-glycosylated human interleukin-2.

6. A method as claimed in Claim 4, wherein the pH is about 3.

7. A method of producing interleukin-2-containing liposome composition by mixing together a phospholipid solution and an interleukin-2-containing buffer soltuion to prepare a W/O type emulsion, gelating this emulsion, then adding a buffer to the resulting gel, which comprises adjusting the pH of at least the buffer to be added to the gel to 2 to 3.5.

8. A method as claimed in Claim 7, wherein the interleukin-2 is a recombinant non-glycosylated human interleukin-2.

9. A method as claimed in Claim 7, wherein the pH is about 3.

## Fig. 1

```
1
Ala Pro Thr Ser Ser Ser Thr Lys Lys Thr Gln Leu Gln

                              20
Leu Glu His Leu Leu Leu Asp Leu Gln Met Ile Leu Asn

Gly Ile Asn Asn Tyr Lys Asn Pro Lys Leu Thr Arg Met
40
Leu Thr Phe Lys Phe Tyr Met Pro Lys Lys Ala Thr Glu

                              60
Leu Lys His Leu Gln Cys Leu Glu Glu Glu Leu Lys Pro

Leu Glu Glu Val Leu Asn Leu Ala Gln Ser Lys Asn Phe
        80
His Leu Arg Pro Arg Asp Leu Ile Ser Asn Ile Asn Val

                              100
Ile Val Leu Glu Leu Lys Gly Ser Glu Thr Thr Phe Met

Cys Glu Tyr Ala Asp Glu Thr Ala Thr Ile Val Glu Phe

        120
Leu Asn Arg Trp Ile Thr Phe Cys Gln Ser Ile Ile Ser

        133
Thr Leu Thr
```

European Patent Office

Application number:

87 310 653. 8

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:     IFO - 14437

FERM  BP - 852

EPO Form 1165 07.81 e